# EUROPEAN PATENT SPECIFICATION

(11) **EP 1 933 912 B1**
(45) Date of publication and mention of the grant of the patent: **24.07.2013**
(21) Application number: 06842335.9
(22) Date of filing: 26.09.2006
(51) Int. Cl.: A61M 16/00

(54) **COMBINED VENTILATOR INEXSUFFLATOR**
KOMBINIERTER VENTILATOR-IN-/EXSUFFLATOR
IN-EXSUFFLATEUR ET VENTILATEUR COMBINES

(30) Priority: 26.09.2005 US 720042 P; 13.07.2006 US 830741 P
(43) Date of publication of application: 25.06.2008
(73) Proprietor: Innovent Medical Solutions, Inc., 91450 Jerusalem (IL)
(72) Inventor: BE'ERI, Eliezer, 97233 Jerusalem (IL)
(74) Representative: HOFFMANN EITLE
(86) International application number: PCT/IB2006/003897
(87) International publication number: WO 2007/054829

(56) References cited:
- CA-A1- 2 230 622
- US-A- 4 448 192
- US-A- 5 303 699
- US-A1- 2005 005 936
- US-A1- 2005 051 174

## Description

### Field of the Invention

The present invention relates to the field of respiratory devices. In particular, the present invention relates to an inexsufflation respiratory device to assist in the removal of pulmonary secretions from airways.

### Background of the Invention

For patients with weak respiratory muscles, inspiratory and/or expiratory devices may be used to assist with inspiration and/or expiration. For example, mechanical ventilators may apply air under pressure to a patient during inhalation to facilitate respiration. For patients with a weak cough, assistance with coughing during expiration can protect against infection by removing airway secretions from the lungs and air passages. Patients on mechanical ventilation in an intensive care unit may require frequent secretion removal treatments so as to keep their airways free of respiratory secretions. Several methods for performing secretion removal in ventilated patients are known in the art.

The most common secretion removal method currently known in the art is invasive catheter suction, in which a narrow-gauge catheter is inserted into the patient's airways via an endotracheal or tracheostomy tube, and continuous suction is applied as the catheter is withdrawn from the patient. If the catheter comes into close proximity with the secretions, the secretions adhere to, or are sucked into, the catheter, and are removed as the catheter is withdrawn from the body. However, drawbacks of this method include its invasive nature and the potential for scarring of the airways due to the insertion and removal of the catheter.

Another method for secretion removal employed as an alternative to catheter suction is known as mechanical inexsufflation (MIE). In mechanical inexsufflation, the lungs are first insufflated to near maximum vital capacity, and then rapidly and suddenly exsufflated by sucking air out of the lungs at a high velocity. Because air is expelled from the airways at high velocity, the airflow carries secretions up and out of the lungs with the high velocity air flow. Mechanical inexsufflation thus removes airway secretions by simulating a natural cough. Mechanical inexsufflation may be performed using a facemask, endotracheal tube, tracheostomy or other suitable patient interface.

Mechanical inexsufflation may be preferred over catheter suction due to its non-invasive nature. In addition, mechanical inexsufflation generates airflow within the entire diameter and length of the patient's functional airway and at a high flow rate, thus causing expulsion of secretions from the entire airway. In contrast, catheter suction generates airflow only within the narrow suction catheter, and at a relatively lower flow rate. Because of its physical dimensions, when a suction catheter is inserted into the airways it is capable of reaching only the larger, more proximal airways, but not the small, distant, more peripheral airways. In addition, the branching morphology of the left and right bronchi is such that suction catheters usually enter the right mainstem bronchus, and usually miss the left mainstem bronchus, when the catheter is inserted into the airways. During catheter suction, much of the patient's functional airway is therefore not exposed to the catheter and suction airflow, and consequently little or no removal of secretions from those areas occurs.

In mechanical inexsufflation, secretions are physically removed by airflow within the patient's airway, whereas in catheter suction, secretions are physically removed by the catheter itself.

Several devices for performing secretion removal via a suction catheter in combination with a source of positive gas pressure are known in the art. For example, devices known in the art may connect a source of positive pressure, such as an oxygen cylinder, and a source of negative pressure, such as a suction device, with a suction catheter for purposes of lung insufflation (with oxygen) and secretion removal through the suction catheter. Such devices are unsuitable for performing mechanical inexsufflation because the narrow diameter of the suction catheter precludes the generation of an exsufflatory flow equivalent to that of a natural cough, about 160 liters per minute in an adult, or about 14 liters per minute in an infant, as desired.

For example, a suction catheter generally has an inner diameter of between about one millimeter and about two millimeters, whereas the natural airway of an adult patient, or an endotracheal tube inserted into an adult patient, typically has an inner diameter of between about five millimeters and about ten millimeters. As gas flow rate is proportional to the diameter of the channel through which the gas flows, the larger diameter airflow channel through which flow is generated in mechanical inexsufflation allows commercially available suction devices to generate gas flow rates that approximate those of a nature cough (i.e., about 160 liters per minute at a pressure gradient of 60 cm H₂O), whereas the smaller diameter airflow channel through which catheter suction is performed precludes generation of cough flow rates, and generally results in low flow (less than about two liters per minute at a pressure gradient of 200 cm H₂O). Attempts to generate higher flow through a suction catheter by increasing the suction force several-fold could cause the soft plastic suction catheter to collapse and cut off flow completely. In addition, the length of suction catheters (usually about 60cm) is much longer than the length of endotracheal or tracheostomy tubes (usually about 10cm to 25cm). The combination of increased length and decreased diameter results in a much higher resistance to airflow through a suction catheter than through an endotracheal or tracheostomy tube. Therefore, use of a suction catheter as an exsufflatory airflow channel to remove airway secretions by cough simulation is generally difficult, if possible at all, inefficient, or otherwise undesirable.

In addition, use of an oxygen cylinder as a source of positive pressure gas flow for a cough insufflation has drawbacks. It is preferable in cough simulation that the preceding insufflation be carefully measured so as to ensure that the patient's maximal lung vital capacity (i.e. the maximum volume to which the lung can be safely inflated) has been reached, but not exceeded. The cough will generally be less effective if the vital capacity is not reached before exsufflation commences, because there will not be enough air in the lungs to blow secretions out of the airways. In addition, if the vital capacity is exceeded before exsufflation commences, a pneumothorax may ensure, damaging the patient's lungs. Careful calibration of the insufflatory airflow volume may thus be desirable for the effective and safe performance of mechanical inexsufflation. An oxygen cylinder usually lacks a mechanism for calibrating the volume of gas leaving the cylinder, and may therefore be a dangerous or ineffective method for achieving lung insufflation to the patient's vital capacity.

An example of a conventional inexsufflator is described in U.S. Patent Number 7,096,866, issued August 29, 2006 and entitled "Inexsufflator".

Another device known and used in the prior art for performing mechanical inexsufflation is the "CoughAssist®" from the JH Emerson Company of Cambridge, MA. The CoughAssist® device uses a turbine to perform insufflation of the lungs by blowing air into a patient at a defined pressure for a predetermined period of time through a tubing connected to the patient's endotrachael tube, tracheostomy tube or facemask. After the predetermined period of time, a valve mechanism within the CoughAssist® device rapidly switches the direction of airflow within the length of tubing, resulting in rapid exsufflation of the patient's lungs. The exsufflation flow continues until the valve mechanism disconnects the tubing from the turbine, terminating the exsufflation flow. There is then a pause period, during which no airflow occurs and airway pressure is equal to zero (atmospheric pressure), until the next insufflation cycle commences. This pause period is necessary to avoid hyperventilation of the patient, and usually lasts about one to three seconds. The cycle is repeated several times to complete the secretion removal treatment.

The CoughAssist® device also suffers from several disadvantages. For example, the CoughAssist® device requires a patient to be disconnected from a medical ventilator to perform the mechanical inexsufflation procedure. Disconnection from a medical ventilator in order to connect the patient to the CoughAssist® device may be undesirable, particularly for critically ill patients, who may deteriorate when disconnected from the medical ventilator. The CoughAssist® device also employs a time-cycled cycling mechanism to terminate the phase of inhalation, which may present additional disadvantages, because volume-cycled or flow-cycled cycling mechanisms are usually the safest and most efficient methods for ventilating adults. Furthermore, the CoughAssist® device cannot maintain positive end expiratory pressure (PEEP) during the pause period prior to onset of the next inhalation. PEEP is supra-atmospheric pressure in the airways during the period of expiration, and is often used in intensive care units to manage patients undergoing mechanical ventilation, because it prevents collapse of the lung tissue (atelectasis) and encourages secretion removal. With the CoughAssist® device, however, airway pressure equilibrates with atmospheric pressure during the pause period after exsufflation has ended and before inhalation has started. The CoughAssist® device also does not include alarm systems and other components used in life-support devices.

Another disadvantage of performing mechanical inexsufflation with the CoughAssist® device is that the same tubing carries both exsufflatory airflow and insufflatory airflow between the patient interface and the CoughAssist® device. Exsufflatory airflow contains airway secretions within it, which may be infected. These infected secretions are deposited in the CoughAssist® tubing, through which the insufflatory airflow of the next treatment cycle passes. Insufflation through the same tubing that has just been used for exsufflation therefore carries a risk of causing immediate reinfection of the lungs from which the secretions were cleared. Moreover, because the same turbine is used for generating both insufflation and exsufflation airflows, the turbine is exposed to potentially infected airway secretions, potentially limiting the lifetime of the turbine and creating a hazard of infecting a different patient who may use the same CoughAssist® machine later.

US 4,448,192 discloses a ventilator which accommodates to a patient's efforts in breathing and to his change in respiratory requirements by a system with an allegedly novel control law. Said allegedly novel control law determines a target pressure-volume wave-form which characterizes the patient's respiratory requirements; this waveform is to be constantly modified and corrected during the course of the ventilator operation.

US 2005/0051174 discloses an insufflation-exsufflation system with percussive assist for removal at broncho-pulmonary secretions. Said system comprises a blover having a positive and negative outputs for insufflation and exsufflation respectively, and a three position slide valve connecting a patient interface alternatively to the positive or negative outputs of the blover.

US 2005/0005936 A describes methods, systems and devices for ventilation in which specific lung areas are ventilated with an indwelling trans-tracheobronchial catheter. Trans-Tracheobronchial Segmental Ventilation (TTSVP) is performed on either a naturally breathing or mechanical ventilated patient by placing a specifically configured indwelling catheter into a bronchus of a poorly ventilated specific lung area and providing direct ventilation to that area. Ventilation includes delivery of respiratory gases, therapeutic gases or agents and evacuation of stagnant gases, mixed gases or waste fluids.

### Summary of the Invention

The present invention provides a mechanical inexsufflation device for assisting with respiration, coughing and/or secretion removal in a patient according to claim 1. The illustrative mechanical inexsufflation device of the present invention includes a medical ventilator or other suitable device for conveying airflow under positive pressure, a first gas flow channel connected to the medical ventilator and operative to convey unidirectional gas flow, a first gate operative to selectively open or obstruct gas flow through the first gas flow channel, and a source of negative pressure gas flow, which is preferably capable of conveying unidirectional gas flow at a flow rate of at least fourteen liters per minute. The source of negative pressure gas flow is preferably capable of generating negative pressure simultaneously with the generation of airflow under positive pressure by a source of positive pressure in the ventilator. A second gas flow channel connected to the source of negative pressure gas includes a second gate that may selectively open or obstruct gas flow through the second gas flow channel. A control unit operates to open or close the first and second gates in a mutually reciprocal and opposite manner. A patient interface unit conducts airflow to and from a patient's lungs according to the settings of the gates.

The mechanical inexsufflation device preferably does not include a valve mechanism connected to the endotracheal tube, which provides advantages over prior respiratory devices. In addition, the valve mechanism may be lightweight and/or smaller than valve mechanisms of the prior art. The mechanical inexsufflation device may include ventilation tubing connected directly to a patient interface and a suction unit connected to a patient interface using a suitable tubing or other connection means.

According to a first aspect of the invention, a mechanical inexsufflation device is provided. The mechanical inexsufflation devices comprises a patient interface unit configured to permit a negative pressure airflow therethrough and a positive pressure airflow from a medical mechanical ventilator, a suction unit for generating airflow under negative pressure, a first valve for selectively blocking airflow from the medical mechanical ventilator and a second valve separate from the first valve for selectively blocking airflow to the suction unit.

The mechanical inexsufflation device also includes a medical mechanical ventilator connected to the patient interface unit for generating airflow under positive pressure

The patient interface unit is configured to permit a negative pressure airflow of between about 14 liters per minute and about 800 liters per minute (i.e., the flow rate range of a natural cough). In a specific embodiment, airflow is in the range of from about 14 liters per minute to about 500 liters per minute.

### Brief Description of the Figures

Figure 1 is a schematic diagram of a mechanical inexsufflation device according to an illustrative embodiment of the invention;
Figure 2 illustrates a tubing suitable for use in the mechanical inexsufflation device of Figure 1;
Figure 3 is a flow chart illustrating the steps involved in performing a mechanical inexsufflation using a mechanical inexsufflation device of an illustrative embodiment of the invention;
Figure 4 is a schematic diagram of a mechanical inexsufflation device according to an illustrative embodiment of the invention;
Figure 5 is a flow chart illustrating the steps involved in performing a mechanical inexsufflation using the mechanical inexsufflation device of Figure 4.

### Detailed Description of the Invention

The present invention provides an improved mechanical insufflation-exsufflation (i.e., inexsufflation) device for performing mechanical inexsufflation to remove secretions from a patient's lungs. The present invention will be described below relative to certain illustrative embodiments. Those skilled in the art will recognize that the invention is not limited to the illustrative embodiments, and may include certain changes and variations.

As used herein, the term "insufflation", and the like, refers to the blowing of air, vapor, or a gas into the lungs of a patient.

As used herein, the term "exsufflation", and the like, refers to the forced expiration of air, vapor or gas from the lungs of a patient.

The illustrative mechanical inexsufflation device of the present invention comprises a device for generating airflow under positive pressure, such as a medical ventilator, a first gas flow channel connected to the medical ventilator and operative to convey unidirectional gas flow, a first gate operative to selectively open or obstruct gas flow through the first gas flow channel, and a source of negative pressure gas flow, which is preferably capable of conveying unidirectional gas flow at a flow rate of at least fourteen liters per minute. The source of negative pressure gas flow preferably generates negative pressure simultaneously with the source of positive pressure in the ventilator. A second gas flow channel connected to the source of negative pressure gas includes a second gate that may selectively open or obstruct gas flow through the second gas flow channel. A control unit operates to open or close the first and second gates in a mutually reciprocal and opposite manner. A patient interface unit conducts airflow to and from a patient's lungs to perform insufflation or exsufflation, depending on the operation of the gates within the flow channels.

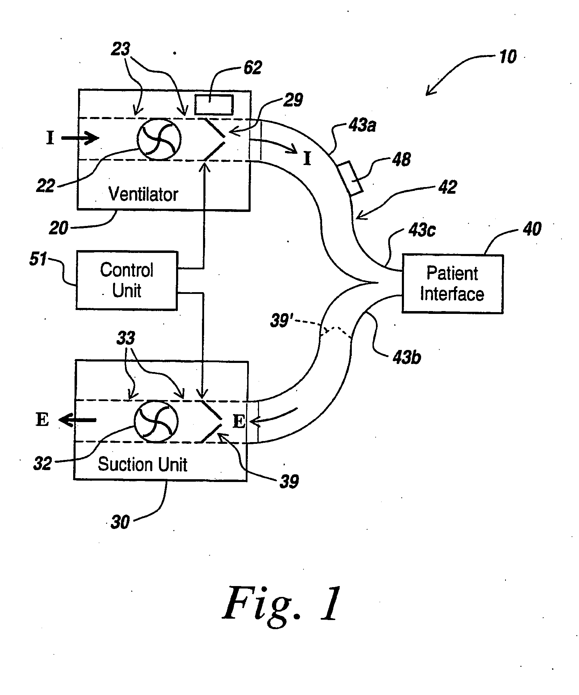

Referring to Figure 1, a mechanical inexsufflation device 10 of an illustrative embodiment of the invention includes a mechanical medical ventilator 20 for generating airflow under positive-pressure or another source of positive pressure airflow. The positive pressure airflow may be used for insufflation of a patient. The illustrative mechanical ventilator 20 has a positive-pressure airflow generator 22, such as a turbine, piston, bellow or other devices known in the art, for generating airflow under positive pressure. One skilled in the art will recognize that the airflow generator 22 may be any suitable device or mechanism for generating positive pressure airflow and is not limited to the above-mentioned devices. An inflow airflow channel 23 is connected to an inlet and outlet of the generator 22 to convey and supply gas flow from the airflow generator 22. The direction of airflow through the airflow generator 22 and the associated airflow channel 23 is illustrated by the arrows labeled "I".

The illustrative ventilator 20 for generating airflow under positive-pressure may be any suitable ventilator and is not limited to a particular type of medical ventilator. For example, the device may be a standard volume-cycled, flow-cycled, time-cycled or pressure-cycled life support or home use medical ventilator, or any medical ventilator or other device capable of generating positive end expiratory pressure (PEEP). Such devices are known in the art.

The ventilator 20 for generating airflow under positive-pressure preferably includes a calibration means 62 for calibrating the insufflatory airflow, as is standard practice in all medical ventilators. This calibration means 62 is also known as the "cycling mechanism" of the ventilator, and may operate on the basis of volume-cycled, flow-cycled, time-cycled or pressure-cycled mechanisms of calibration, or other basis known in the art.

The illustrative mechanical inexsufflation device 10 further includes a suction unit 30 for generating airflow under negative pressure, which may be used to perform exsufflation of a patient. The illustrative suction unit 30 includes a negative-pressure airflow generator 32 for generating a suction force, and an outflow airflow channel 33 for conveying airflow to and through the negative-pressure airflow generator 32 under negative pressure. The negative pressure airflow generator 32 may be any suitable device or mechanism for generating negative pressure airflow, including, but not limited to, a turbine, piston, bellow or other devices known in the art. The direction of airflow to the airflow generator 32 and through the associated airflow channel 33 is illustrated by the arrows E.

A patient interface unit 40 interfaces the suction unit 30 and medical ventilator 20 with a patient. As shown, the inflow airflow channel 23 and outflow airflow channel 33 are connected to the patient interface unit 40 by means of tubing 42 or other suitable means. The illustrative patient interface unit 40 may be an endotrachael tube, a tracheostomy tube, a facemask or other suitable means known in the art for establishing an interface between a patient and another medical device, such as a ventilator or suction unit. The patient interface unit 40 is of sufficient caliber to permit airflow at a flow rate that is substantially equivalent or in the range of the flow rate of a natural cough (generally corresponding to a flow rate of at least about 160 liters per minute through an endotracheal tube of internal diameter of about ten millimeters or about fourteen liters per minute through an endotracheal tube of about three millimeters internal diameter.) The patient interface unit is configured to permit a negative pressure airflow therethrough of at least 14 liters per minute, ranging up to about 800 liters per minute, which covers the range of cough flow rates from infants to adults. In a specific embodiment, the airflow is in the range of from about 14 to about 500 liters per minute. The patient interface unit is also configured to permit positive pressure airflow from a medical mechanical ventilator. The device 10 may include a sensor 48, illustrated as a component on the tubing 42 between the interface 40 and the gate 29, for detecting an inspiratory pressure generated by the device, particularly a peak inspiratory pressure, as described below. The sensor may alternatively be located in any suitable location relative to the patient. For example, the sensor 48 may alternatively be located between the interface 40 and the gate 39, or within the ventilator 40.

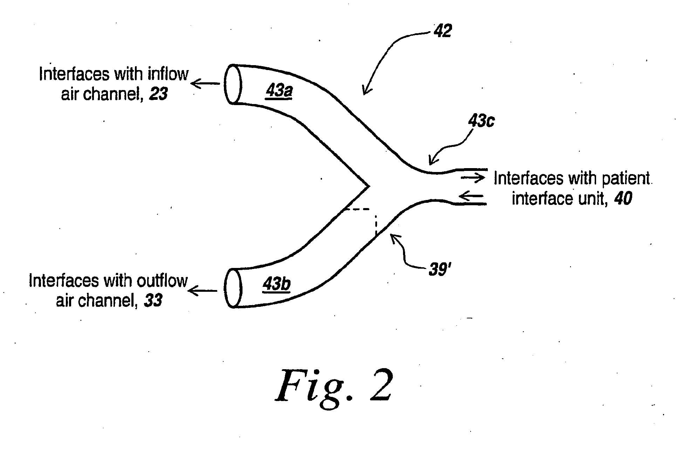

The illustrative tubing 42, illustrated in detail Figure 2, may be a standard twenty-two millimeter diameter ventilator tubing or other suitable tubing known in the art. The tubing 42 preferably is substantially branched, having two limbs 43a, 43b, each of which connects with air channels 23 and 33, respectively. The illustrative tubing is y-shaped, though the tubing may alternatively be t-shaped or have any other suitable shape known in the art. The ends of the limbs 43a and 43b may connect to and interface with the air channels through any suitable means known in the art, such as friction fit and other connection means. The limbs 43a, 43b may extend at any suitable angle relative to a main portion 43c of the tubing. As shown, the main portion 43c of the tubing connects to the patient interface 40 through any suitable means known in the art.

Alternatively, the tubing 42 may comprise a single length of double-lumen tubing, with the two lumens joining together at the point of connection to the patient interface unit 40. One skilled in the art will recognize that any suitable means may be used for connecting both the ventilator 20 and the suction unit 30 to the patient interface unit 40. For example, two lengths of non-intersecting tubing coupled between the patent interface 40, the ventilator 20 and the suction unit 30.

Each airflow channel 23, 33 may include a valve, illustrated as gates 29, 39, respectively, for regulating airflow through the corresponding airflow channel. Each gate 29, 39 may selectively form a physical barrier to airflow within the corresponding airflow channel. Each gate 29, 39 may be selectively opened, to allow air to flow unobstructed through the corresponding airflow channel, or closed to block the corresponding airflow channel. For example, when gate 29 is open, positive pressure airflow generated by the medical ventilator 20 is delivered to the patient interface unit via channel 23 and tubing portions 43a, 43c. When gate 39 is open, negative pressure airflow generated by the suction unit 30 is permitted to flow from the patient interface device 40 to and through the suction unit 30 via tubing portions 43c, 43b and channel 33. The gates 29, 39 may comprise any suitable means for allowing reversible closing and opening of an airflow channel, including, but not limited to, membranes, balloons, plastic, metal or other mechanisms known in the art.

The inflow gate 29 or other valving means for selectively opening and closing the inflow airflow channel 23 may be located in any suitable position along the inflow airflow path. In one embodiment, the gate 29 may be located at any location along the inflow airflow channel 23 within the ventilator. The gate may be located at the air outlet of the ventilator in the inflow airflow channel 23 or in another location. Alternatively, the inflow gate 29 may be located within the tubing 42, such as in the limb 43a and illustrated in phantom as inflow gate 29'. The outflow gate 39 is preferably located between the outflow airflow generator 32 and the patient interface unit 40. In one embodiment, the outflow gate 39 is located at the air inlet of the suction device 30.

Alternatively the outflow gate may be located in the tubing 42, such as in the limb 43b. The alternative embodiment of the outflow gate 39' is shown in phantom in Figures 1 and 2.

A control unit 51 controls the operation of the gates 29 and 39. The control unit 51 may comprise a microprocessor running software algorithms receiving inputs from pressure sensors, flow sensors and the control panels of the ventilator 20 and suction unit 30. The output of the microprocessor in the control unit 51 may connect to the electronic circuitry of the ventilator 20 and suction unit 30, as well as to the gates 29 and 39 to control and coordinate the operation of these components, as described below.

The illustrative mechanical inexsufflation device may be formed by retrofitting an existing medical ventilator with a suction unit 30, patient interface unit 40 and/or tubing 42 capable of selectively connecting both the suction unit and ventilator to the patient interface. Alternatively, a patient interface unit 40 with appropriate tubing 42 may be provided for retrofitting a suction unit and medical ventilator to perform mechanical inexsufflation.

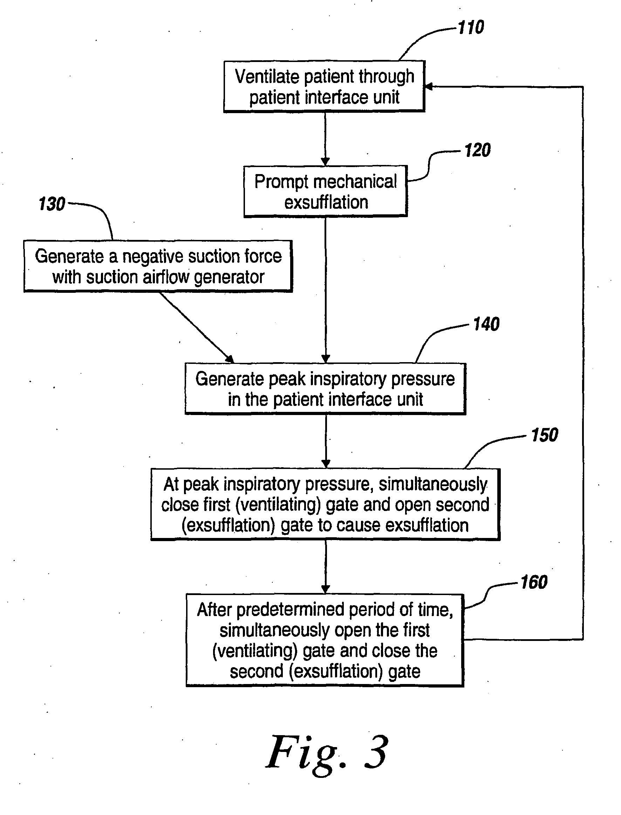

Figure 3 illustrates the steps involved in operating the mechanical inexsufflation device 10 according to an illustrative embodiment of the invention. In a first step 110, the mechanical inexsufflation device 10 is in a resting state, in which the ventilator 20 ventilates a patient through the patient interface unit 40. In the resting state, the first gate 29 in the inflow airpath, defined by airflow channel 23 and limb 42a, is open to allow positive pressure airflow generated by the generator 22 through the inflow airpath under positive pressure, while the second gate 39 in the outflow airpath, defined by outflow channel 33 and limb 42b is closed to prevent airflow through the outflow airpath. The device remains in the first state, continuously ventilating the patient, until secretion removal by mechanical inexsufflation is desired or prompted.

When mechanical inexsufflation is prompted in step 120, the control unit 51 prepares to apply negative pressure airflow to the lungs to effect secretion removal. To effect secretion removal, the control unit switches on, if not already on, the suction airflow generator 32 such that the suction airflow generator 32 then generates a negative suction force in step 130. Preferably, in step 130, the suction airflow generator produces a pressure differential of 30 to 130 and preferably approximately 70 cm H₂0 in comparison to the maximum pressure in the patient interface unit 40 during ongoing ventilation in step 120. In one embodiment, the suction airflow generator generates a suction force after mechanical inexsufflation is prompted in step 120. Alternatively, the suction airflow generator may generate a negative pressure airflow even before prompting of the mechanical inexsufflation in step 120, such that suction force is in effect while or even before ventilation occurs in step 110. Steps 120 and 130 may be incorporated into a single step, involving powering on a suction unit in preparation for performing secretion clearance, if the suction unit is not already powered on.

The switch to initiate mechanical inexsufflation may be triggered by an input from the control panel of the ventilator 20, from a pressure sensor in the ventilator 20, or prompted by a timing mechanism in the control unit 51, or by a mechanical prompting by a user. During step 130, when the suction force is initiated, the outflow gate 39 remains closed, so that the patient interface unit 40 is not exposed to the suction force being generated. During step 130, positive pressure continues to be generated by the ventilator 20 simultaneously with the generation of negative pressure by the suction airflow generator 32.

The conditions of step 130 continue until the ventilator 20 generates a peak inspiratory pressure in the patient interface unit 40 in step 140. The peak inspiratory pressure may be detected by a sensor 48, which then signals the control unit 51, or other suitable means. The use of a ventilator, which has means to measure and calibrate an insufflation, ensures that a patient's maximal lung vital capacity is reached, but not exceeded, to promote effective secretion removal.

When peak inspiratory pressure is reached, the control unit 51 closes the first, ventilating, gate 29 and simultaneously opens the second, exsufflatory, gate 39 in step 150. The switching between the gates while both airflow generators 22 and 32 are operating rapidly and suddenly exposes the patient to the pressure gradient generated by the suction airflow generator 32, and exsufflation of air from the lungs towards the suction unit 30 ensues. In the illustrative embodiment of the present invention, the simultaneous or near simultaneous closure of the first gate 29 ensures that the negative pressure generated by the suction airflow generator 32 does not suck atmospheric air in through the inflow airflow channel 23.

After a predetermined time period, which may be between about one and about two seconds or any suitable interval, the control unit 51, in step 160, causes the second, exsufflatory, gate 39 to close, and the first, ventilating, gate 29 to simultaneously open. The suction unit 30 may be switched off after sealing the outflow airpath, or may continue to operate without affecting the subsequent ventilation by the ventilator 20.

Throughout steps 120 through 160, the ventilator 20 preferably continues operating continuously, including during the period of time that gate 29 is closed. Thus, immediately upon opening of gate 29, the patient is exposed to the ongoing positive pressure ventilation cycle of ventilator 20. The ventilator 20 then ventilates the patient through the patient interface unit 40 as in step 110, during a "pause" period until the control unit 51 initiates another mechanical inexsufflation cycle in step 120, and the illustrated steps 120-160 are repeated. During the pause period between mechanical inexsufflations, the patient receives full ventilation, according to all the ventilator's ventilation parameters (including provision of PEEP and enriched oxygen).

The control unit 51 in the illustrative device may also control other aspects of the electronic and mechanical functioning of the ventilator 20 and the suction unit 30 before, during and after a mechanical inexsufflation treatment. For example, the control unit 51 may override the normal alarm functions of the ventilator 20 so as to prevent the alarms from sounding because of high pressure detected proximal to the closed gate 29.

Alternatively, the control unit 51 may cause an increased tidal volume to be delivered to the patient in the breath immediately prior to an exsufflation to facilitate the exsufflation process.

The control unit 51 may also or alternatively be programmed to initiate a cycle of mechanical inexsufflation treatment in step 120 whenever a control button on the ventilator 20 is activated, or whenever a high intrathoracic pressure is detected in the patient using a sensor. Alternatively, the control unit 51 may be programmed to initiate step 120 and a subsequent cycle of mechanical inexsufflation treatment at a predetermined frequency.

In another embodiment, the control unit 51 may adjust the timing of the insufflation and exsufflation cycles, as well as, or alternatively, the strength of the positive pressure and negative pressure airflow used in the mechanical inexsufflation treatments.

The control unit 51 may be located within the ventilator 20, the suction unit 30 or in any suitable location to effect control of various components of the device 10. The control unit 51 can communicate with the ventilator unit 20, the suction unit 30 or both in either a wired or wireless manner.

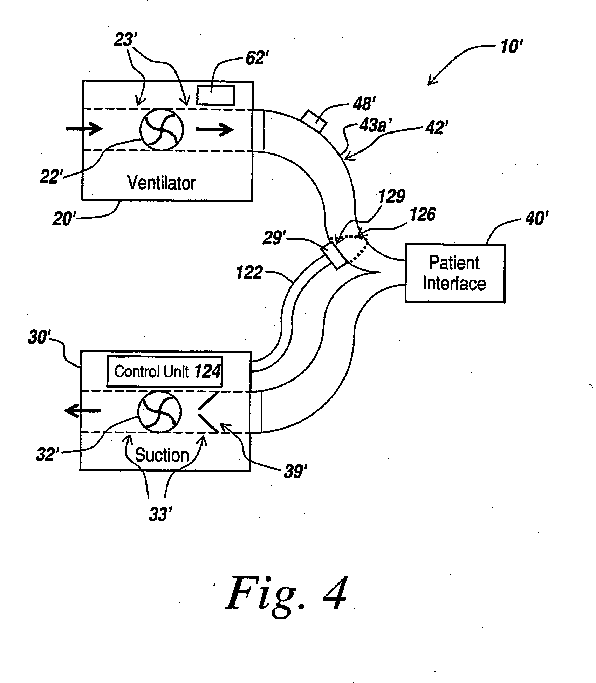

Figure 4 is a schematic drawing of a mechanical inexsufflation device 10' according to another embodiment of the invention. In the embodiment of Figure 4, the gate 29' within the inflow airflow path comprises a pneumatically-activated member, illustrated as a pneumatically-activated membrane 129. In the illustrative embodiment, the gate 29' is disposed within the first, inflow, branch 43a' of the tubing 42', though the gate 29' may alternatively be disposed in another suitable location within the device 10'. During operation of the device 10', in the resting, ventilating state of step 110, the membrane 129 is substantially flat and does not obstruct the lumen of the tubing 42'. A pneumatic mechanism 122 is in communication with the membrane 129 and suction unit 30'. A control unit 124 controls the activation and deactivation of the second gate 39' and membrane 129 of the first gate 29'. In this embodiment, the control unit 124 does not receive inputs from, or have outputs to the electronic circuitry of the ventilator 20'. When the gate 29' is activated, the pneumatic mechanism 122 generates an increase in pneumatic pressure behind the membrane 129, causing the membrane 129 to bulge and thereby obstruct the lumen of the tubing, as illustrated by the dotted line 126. In an alternative embodiment of device 10', the gate 29' may comprise a pneumatically activated piston, or any other pneumatically activated valve mechanism. Reference numerals 22', 23', 48' and 62' refer to elements corresponding to the elements 22, 23, 48 and 62, respectively, as explained in relation to Figure 1.

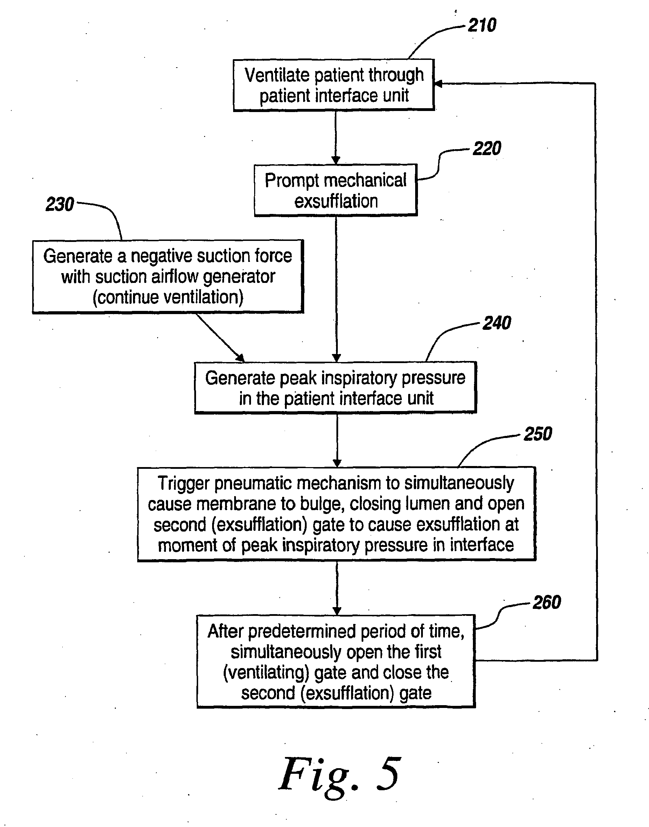

Figure 5 illustrates the steps involved in operating the device 10' of Figure 4 to perform mechanical inexsufflation cycles. In a resting state in step 210, the device 10' ventilates a patient through the patient interface unit 40', with the gate 29' open and the gate 39' closed. When secretion removal by mechanical inexsufflation is desired, a prompt may be given in step 220. In step 230, which may occur before, during or after step 110 and/or step 220, the operator powers on the suction unit 30', such that the airflow generator 32' generates a negative suction force, producing a pressure differential of 30 to 130 and preferably about 70 cm H₂O in comparison to the maximum pressure in the interface unit 40' during ongoing ventilation. Steps 220 and 230 may be incorporated into a single step, involving powering on a suction unit in preparation for performing secretion clearance, if the suction unit is not already powered on. Because the second gate 39' is closed during steps 220 and 230, the patient interface unit 40' is not initially exposed to the suction force in step 230. In step 230, the ventilator 20' continues to generate cycles of positive pressure simultaneous with the generation of negative pressure by the airflow generator 32'. In step 240, as the ventilator generates a peak inspiratory pressure in the patient interface unit 40', the operator initiates the control unit 124 to cause the pneumatic mechanism 122 to activate the gate 29' and to simultaneously open the second gate 39' in step 250. The operator my prompt the control unit 124 by pressing a button on the control panel of the suction unit 30', or through other suitable means. As a result of the actions in step 250, the patient is suddenly and rapidly exposed to the pressure gradient generated by the airflow generator 32', and exsufflation of air from the lungs and towards the suction unit 30' ensues in step 250. The closure of the first gate 29' ensures that the negative pressure generated by the airflow generator 32' does not suck atmospheric pressure in through the airflow channel 33'. In step 260, the control unit 124 simultaneously causes the second gate 39' to close and the first gate 29' to open. Step 260 may be initiated after a predetermined time-period, such as between about one and about two seconds, or after any suitable time. Then, the cycle returns to step 210, in which the ventilator 20' ventilates the patient through the patient interface unit 40' as before, until the control unit 124 initiates another mechanical inexsufflation cycle.

The mechanical inexsufflation device of the illustrative embodiments of the invention provide significant advantages over the prior art. For example, compared to traditional catheter suctioning for secretion removal, the mechanical inexsufflation device provides decreased mucosal trauma, increased patient comfort and greater efficiency. Compared to other inexsufflation devices, the current mechanical inexsufflation device preferably does not include a valve mechanism connected directly to the endotracheal tube, which frees the endotracheal tube from the weight of a valve, reducing the risk of accidental intubation and making the patient's respiratory tubing easier to manage. In addition, the valve mechanism may be lightweight and/or smaller than valve mechanisms of the prior art, facilitating automation of the coordinating valves. These factors reduce the risk of accidental decannulation caused by the weight of the valve mechanism on the endotracheal tube or tracheostomy, as well as reducing the risk of sudden extubation. The configuration of the device facilitates automatic or semi-automatic operation of the device, in particular, the valves, which may optimize its efficacy.

Compared to the CoughAssist® device, the current invention does not require disconnecting the ventilated patient from his ventilator so as to perform inexsufflation. Therefore, the patient continues to receive essential ventilator parameters, such as PEEP provided by the ventilator, during the pause period between each inexsufflation cycle. As PEEP facilitates secretion removal, this is a distinct advantage as compared with the CoughAssist® device.

The present invention has been described relative to certain illustrative embodiments. Since certain changes may be made in the above constructions without departing from the scope of the invention, it is intended that all matter contained in the above description or shown in the accompanying drawings be interpreted as illustrative and not in a limiting sense. It is also to be understood that the following claims are to cover all generic and specific features of the invention described herein, and all statements of the scope of the invention which, as a matter of language, might be said to fall therebetween.

## Claims

1. A mechanical inexsufflation device (10), comprising:
a patient interface unit (40) configured to permit a negative pressure airflow of between about 14 litres per minute and about 800 litres per minute therethrough, and a positive pressure airflow from a medical mechanical ventilator or other suitable device for conveying airflow under positive pressure;
a suction unit (30) for generating airflow under negative pressure that flows through the patient interface unit;
a medical mechanical ventilator (20) or other suitable device for conveying airflow under positive pressure connected to the patient interface unit for generating the positive pressure airflow;
a first valve (29) for selectively blocking the airflow from the medical mechanical ventilator or other suitable device for conveying airflow under positive pressure to the patient interface unit; and
a second valve (39) for selectively blocking the airflow from the patient interface unit to the suction unit; wherein
the suction unit is capable of generating negative pressure airflow simultaneously with the generation of positive pressure airflow by the medical mechanical ventilator or other suitable device for conveying airflow under positive pressure, **characterised in that** the second valve (39) is separate from the first valve.

2. The mechanical inexsufflation device of claim 1, further comprising a tubing (42) for connecting the medical mechanical ventilator or other suitable device for conveying airflow under positive pressure, and the suction unit to the patient interface unit.

3. The mechanical inexsufflation device of claim 2, wherein the tubing comprises a main portion (43c) for connecting the patient interface unit, a first limb (43a) for connecting the main portion to the medical mechanical ventilator or other suitable device for conveying airflow under positive pressure, and a second limb (43b) for connecting the main portion to the suction unit.

4. The mechanical inexsufflation device of claim 3, wherein the first valve comprises a pneumatically-activated membrane (129).

5. The mechanical inexsufflation device of claim 4, wherein the pneumatically-activated membrane is disposed within the first limb and lays flat within a lumen of the first limb when the first valve is not activated to allow flow through the first limb.

6. The mechanical inexsufflation device of claim 5, further comprising a pneumatic mechanism (122) in communication with the suction unit and the pneumatically-activated membrane for selectively activating first valve by causing the pneumatically-activated membrane to bulge and block the lumen of the first limb of the tubing.

7. The mechanical inexsufflation device of claim 6, wherein the first valve is located within an airflow channel (23) in communication with the medical mechanical ventilator or other suitable device for conveying airflow under positive pressure.

8. The mechanical inexsufflation device of claim 1, wherein the second valve is located within an airflow channel (33) in the suction unit.

9. The mechanical inexsufflation device of claim 1, further comprising a control unit (51) for controlling operation of the first valve and the second valve.

10. The mechanical inexsufflation device of claim 9, wherein the control unit is configured to simultaneously open the second valve and close the first valve to effect exsufflation of a patient's lungs.

11. The mechanical inexsufflation device of claim 10, wherein the control unit simultaneously opens the second valve and closes the first valve to effect exsufflation of a patient's lungs when a peak inspiratory pressure is generated by the medical mechanical ventilator or other suitable device for conveying airflow under positive pressure, in the patient interface unit connecting the medical mechanical ventilator or other suitable device for conveying airflow under positive pressure, and the suction unit to the patient.

12. The mechanical inexsufflation device of claim 10, wherein the control unit is configured to simultaneously close the second valve and open the first valve to cease exsufflation of a patient's lungs.

13. The mechanical inexsufflation device of claim 9, wherein the control unit controls operation of the medical mechanical ventilator or other suitable device for conveying airflow under positive pressure.

14. The mechanical inexsufflation device according to any one of claims 1 to 8, furthermore comprising a control unit for controlling said mechanical inexsufflation device, having a microprocessor including instructions for performing the computer-implemented steps of:
selectively opening and closing the first valve to allow positive pressure airflow from the medical mechanical ventilator or other suitable device for conveying airflow under positive pressure, to insufflate a patient's lungs; and
selectively opening and closing the second valve to allow negative pressure airflow to exsufflate a patient's lungs.

15. The mechanical inexsufflation device of claim 14, wherein the control unit is programmed to switch on the suction unit to generate the negative pressure airflow when prompted.

16. The mechanical inexsufflation device of claim 14, wherein the control unit is programmed to perform the step of simultaneously opening the second valve and closing the first valve after switching on the suction unit to effect exsufflation.

17. The mechanical inexsufflation device of claim 16, wherein the control unit is programmed to perform the step of increasing a tidal volume of the positive pressure airflow to the patient immediately prior to the step of simultaneously opening the second valve and closing the first valve.

18. The mechanical inexsufflation device of claim 16, wherein the control unit is programmed to perform the step of simultaneously opening the second valve and closing the first valve when a peak inspiratory pressure is reached in a patient.

19. The mechanical inexsufflation device of claim 18, wherein the control unit is programmed to perform the step of simultaneously closing the second valve and opening the first valve after a predetermined period of time to cease exsufflation.

20. The mechanical inexsufflation device of claim 18, wherein the control unit is programmed to trigger the step of simultaneously opening the second valve and closing the first valve from one of: activation of a control button, detection of a high intrathoracic pressure in a patient and after a predetermined interval.

21. The mechanical inexsufflation device of claim 14, wherein the control unit is programmed to override an alarm function of the medical mechanical ventilator or other suitable device for conveying airflow under positive pressure, that generates the positive pressure airflow to insufflate a patient's lungs.

22. The mechanical inexsufflation device of claim 14, wherein the first valve is a pneumatically-activated membrane and the control unit triggers a pneumatic mechanism to generate an increase in pneumatic pressure to selectively close the first valve.

## Patentansprüche

1. Mechanische Inexsufflationsvorrichtung (10), umfassend:
eine Patientenschnittstelleneinheit (40), die dafür eingerichtet ist, Luftfluss mit negativem Druck zwischen etwa 14 Liter pro Minute und etwa 800 Liter pro Minute hindurch zu lassen und einen Luftfluss mit positivem Druck aus einem medizinischen mechanischen Beatmungsgerät oder einem anderen geeigneten Gerät zum Fördern von Luftfluss unter positivem Druck;
einer Saugeinheit (30) zum erzeugen von Luftfluss und negativem Druck, der durch die Patientenschnittstelleneinheit fließt;
ein medizinisches mechanisches Beatmungsgerät (20) oder anderes geeignetes Gerät zum Fördern von Luftfluss unter positivem Druck, das mit der Patientenschnittstelleneinheit verbunden ist, um Luftfluss mit positivem Druck zu erzeugen;
ein erstes Ventil (29) zum selektiven Blockieren des Luftflusses vom medizinischen mechanischen Beatmungsgerät oder dem anderen geeigneten Gerät zum Fördern von Luftfluss unter positivem Druck zur Patientenschnittstelleneinheit; und
ein zweites Ventil (39) zum selektiven blockieren des Luftflusses von der Patientenschnittstelleneinheit zur Saugeinheit; worin
die Saugeinheit in der Lage ist, Luftfluss mit negativem Druck gleichzeitig mit der Erzeugung von Luftfluss mit positivem Druck durch das medizinische mechanische Beatmungsgerät oder das andere geeignete Gerät zum Fördern von Luftfluss unter positivem Druck zu erzeugen, **dadurch gekennzeichnet, dass** das zweite Ventil (39) vom ersten Ventil getrennt ist.

2. Mechanische Inexsufflationsvorrichtung gemäß Anspruch 1, die weiterhin ein Röhrensystem (42) zum Verbinden des medizinischen mechanischen Beatmungsgeräts oder des anderen geeigneten Geräts zum Fördern von Luftfluss unter positivem Druck und der Saugeinheit zur Patientenschnittstelleneinheit umfasst.

3. Mechanische Inexsufflationsvorrichtung gemäß Anspruch 2, worin das Röhrensystem einen Hauptabschnitt (43c) zum Verbinden mit der Patientenschnittstelleneinheit, eine erste Abzweigung (43a) zum Verbinden des Hauptabschnitts mit dem medizinischen mechanischen Beatmungsgerät oder dem anderen Gerät zum Fördern von Luftfluss unter positivem Luftdruck und eine zweite Abzweigung (43b) zum Verbinden des Hauptabschnitts mit der Saueinheit umfasst.

4. Mechanische Inexsufflationsvorrichtung gemäß Anspruch 3, worin das erste Ventil eine pneumatisch aktivierte Membran (129) umfasst.

5. Mechanische Inexsufflationsvorrichtung gemäß Anspruch 4, worin die pneumatisch aktivierte Membran innerhalb der ersten Abzweigung eingebracht ist und flach innerhalb eines Lumens der ersten Abzweigung liegt, wenn das erste Ventil nicht aktiviert ist um Fluss durch die Erste Abzweigung zu erzeugen.

6. Mechanische Inexsufflationsvorrichtung gemäß Anspruch 5, die weiterhin einen pneumatischen Mechanismus (122) in Kommunikation mit der Saugeinheit und der pneumatisch aktivierten Membran zum selektiven Aktivieren des ersten Ventils, in dem die pneumatisch aktivierte Membran dazu gebracht wird, sich zu biegen und das Lumen der ersten Abzweigung der Röhre zu blockieren, umfasst.

7. Mechanische Inexsufflationsvorrichtung gemäß Anspruch 6, worin das erste Ventil innerhalb eines Luftflusskanals (23) in Kommunikation mit dem medizinischen mechanischen Beatmungsgerät oder dem anderen geeigneten Gerät zum Fördern des Luftflusses unter positivem Druck lokalisiert ist.

8. Mechanische Inexsufflationsvorrichtung gemäß Anspruch 1, worin das zweite Ventil innerhalb eines Luftflusskanals (33) in der Saugeinheit lokalisiert ist.

9. Mechanische Inexsufflationsvorrichtung gemäß Anspruch 1, die weiterhin eine Steuerungseinheit (51) zum Steuern der Funktion des ersten Ventils und des zweiten Ventils umfasst.

10. Mechanische Inexsufflationsvorrichtung gemäß Anspruch 9, worin die Steuerungseinheit dafür eingerichtet ist, gleichzeitig das zweite Ventil zu öffnen und das erste Ventil zu schließen, um Exsufflation der Lunge eines Patienten zu bewirken.

11. Mechanische Inexsufflationsvorrichtung gemäß Anspruch 10, worin die Steuerungseinheit gleichzeitig das zweite Ventil öffnet und das erste Ventil schließt, um Exsufflation der Lunge eines Patienten zu bewirken, wenn ein Peak-Atemdruck durch das medizinische mechanische Beatmungsgerät oder das andere geeignete Gerät zum Fördern von Luftfluss unter positivem Druck erzeugt wird, in der Patientenschnittstelleneinheit, die das medizinische mechanische Beatmungsgerät oder das andere geeignete Gerät zum Fördern von Luftfluss unter positivem Druck und die Saugeinheit mit dem Patienten verbindet.

12. Mechanische Inexsufflationsvorrichtung gemäß Anspruch 10, worin die Steuerungseinheit dafür eingerichtet ist, gleichzeig das zweite Ventil zu schließen und das erste Ventil zu öffnen, um die Exsufflation der Lunge eines Patienten zu beenden.

13. Mechanische Inexsufflationsvorrichtung gemäß Anspruch 9, worin die Steuerungseinheit die Arbeit des medizinischen mechanischen Beatmungsgeräts oder anderen geeigneten Geräts zum Fördern von Luftfluss unter positivem Druck steuert.

14. Mechanische Inexsufflationsvorrichtung gemäß irgendeinem der Ansprüche 1 bis 8, die weiterhin eine Steuerungseinheit zum Steuern der mechanischen Inexsufflationsvorrichtung umfasst, die einen Mikroprozessor aufweist, der Instruktionen zum Durchführen der folgenden Computer-implementierten Schritte umfasst:
selektives Öffnen und Schließen des ersten Ventils, um Luftfluss mit positivem Druck von dem medizinischen mechanischen Beatmungsgerät oder anderem geeigneten Gerät zum Fördern von Luftfluss unter positivem Druck zu ermöglichen, um die Lunge eines Patienten zu insufflieren; und
selektives Öffnen und Schließen des zweiten Ventils, um Luftfluss mit negativem Druck zu ermöglichen, um die Lunge eines Patienten zu exsufflieren.

15. Mechanische Inexsufflationsvorrichtung gemäß Anspruch 14, worin die Steuerungseinheit dafür programmiert ist, die Saugeinheit zum Erzeugen von negativem Druck einzuschalten, wenn sie dazu veranlasst wird.

16. Mechanische Inexsufflationsvorrichtung gemäß Anspruch 14, worin die Steuerungseinheit dafür programmiert ist, den Schritt des gleichzeitigen Öffnens des zweiten Ventils und Schließen des ersten Ventils nach dem Einschalten der Saugeinheit durchzuführen, um Exsufflation zu bewirken.

17. Mechanische Inexsufflationsvorrichtung gemäß Anspruch 16, worin die Steuerungseinheit dafür programmiert ist, den Schritt des Steigerns eines Pegelvolumens des Luftflusses mit positivem Druck zum Patienten unmittelbar vor dem Schritt des gleichzeitigen Öffnens des zweiten Ventils und Schließen des ersten Ventils zu bewirken.

18. Mechanische Inexsufflationsvorrichtung gemäß Anspruch 16, worin die Steuerungseinheit dafür programmiert ist, den Schritt des gleichzeitigen Öffnens des zweiten Ventils und Schließen des ersten Ventils zu bewirken, wenn beim Patienten ein Peak-Einatemdruck erreicht ist.

19. Mechanische Inexsufflationsvorrichtung gemäß Anspruch 18, worin die Steuerungseinheit dafür programmiert ist, den Schritt des gleichzeitigen Schließens des zweiten Ventils und Öffnen des ersten Ventils nach einer vorbestimmten Zeitspanne zu bewirken, um die Exsufflation zu beenden.

20. Mechanische Inexsufflationsvorrichtung gemäß Anspruch 18, worin die Steuerungseinheit dafür programmiert ist, den Schritt des gleichzeitigen Öffnens des zweiten Ventils und Schließen des ersten Ventils auszulösen, anhand von einem aus: Aktivierung eines Steuerungsschalters, Detektion eines hohen Intrathorax-Drucks im Patienten und nach einem vorbestimmten Intervall.

21. Mechanische Inexsufflationsvorrichtung gemäß Anspruch 14, worin die Steuerungseinheit dafür programmiert ist, eine Alarmfunktion des medizinischen mechanischen Beatmungsgeräts oder des anderen geeigneten Geräts zum Fördern von Luftfluss unter positivem Druck, das den Luftfluss mit positivem Druck zum Insufflieren der Lunge eines Patienten erzeugt, außer Kraft zu setzen.

22. Mechanische Inexsufflationsvorrichtung gemäß Anspruch 14, worin das erste Ventil eine pneumatisch aktivierte Membran ist und die Steuerungseinheit einen pneumatischen Mechanismus zum Erzeugen eines Anstiegs an pneumatischem Druck zum selektiven Schließen des ersten Ventils auslöst.

## Revendications

1. Dispositif mécanique d'insufflation-exsufflation (10), comprenant :
une unité d'interface patient (40) configurée pour permettre un écoulement d'air sous pression négative d'entre environ 14 litres par minute et environ 800 litres par minute à travers celle-ci, et un écoulement d'air sous pression positive à partir d'un ventilateur mécanique médical ou autre dispositif approprié destiné à transporter un flux d'air sous pression positive ;
une unité d'aspiration (30) destinée à générer un flux d'air sous pression négative qui s'écoule à travers l'unité d'interface patient ;
un ventilateur mécanique médical (20) ou autre dispositif approprié destiné à transporter le flux d'air sous pression positive relié à l'unité d'interface patient pour générer le flux d'air sous pression positive ;
une première soupape (29) pour bloquer sélectivement l'écoulement d'air du ventilateur mécanique médical ou autre dispositif approprié destiné à transporter le flux d'air sous pression positive, à l'unité d'interface patient ; et
une deuxième soupape (39) pour bloquer sélectivement l'écoulement d'air de l'unité d'interface patient à l'unité d'aspiration ; où
l'unité d'aspiration est capable de générer un flux d'air sous pression négative simultanément avec la génération du flux d'air sous pression positive par le ventilateur mécanique médical ou autre dispositif approprié destiné à transporter le flux d'air sous pression positive, **caractérisé en ce que**
la deuxième soupape (39) est séparée de la première soupape.

2. Dispositif mécanique d'insufflation-exsufflation de la revendication 1, comprenant en outre un tuyau (42) pour connecter le ventilateur mécanique médical ou autre dispositif approprié destiné à transporter le flux d'air sous pression positive, et l'unité d'aspiration à l'unité d'interface patient.

3. Dispositif mécanique d'insufflation-exsufflation de la revendication 2, dans lequel le tuyau comprend une partie principale (43c) pour connecter l'unité d'interface patient, une première branche (43a) pour connecter la partie principale au ventilateur mécanique médical ou autre dispositif approprié destiné à transporter le flux d'air sous pression positive, et une deuxième branche (43b) pour connecter la partie principale à l'unité d'aspiration.

4. Dispositif mécanique d'insufflation-exsufflation de la revendication 3, dans lequel la première soupape comprend une membrane à activation pneumatique (129).

5. Dispositif mécanique d'insufflation-exsufflation de la revendication 4, dans lequel la membrane à activation pneumatique est disposée dans la première branche et s'étend à plat dans un lumen de la première branche lorsque la première soupape n'est pas activée pour permettre un écoulement à travers la première branche.

6. Dispositif mécanique d'insufflation-exsufflation de la revendication 5, comprenant en outre un mécanisme pneumatique (122) en communication avec l'unité d'aspiration et la membrane à activation pneumatique pour activer sélectivement la première soupape en amenant la membrane à activation pneumatique à se gonfler et bloquer le lumen de la première branche du tuyau.

7. Dispositif mécanique d'insufflation-exsufflation de la revendication 6, dans lequel la première soupape est située dans un canal d'écoulement d'air (23) en communication avec le ventilateur mécanique médical ou autre dispositif approprié destiné à transporter le flux d'air sous pression positive.

8. Dispositif mécanique d'insufflation-exsufflation de la revendication 1, dans lequel la deuxième soupape est située dans un canal d'écoulement d'air (33) dans l'unité d'aspiration.

9. Dispositif mécanique d'insufflation-exsufflation de la revendication 1, comprenant en outre une unité de commande (51) destinée à commander le fonctionnement de la première soupape et de la deuxième soupape.

10. Dispositif mécanique d'insufflation-exsufflation de la revendication 9, dans lequel l'unité de commande est configurée pour ouvrir la deuxième soupape et fermer la première soupape simultanément afin d'entraîner l'exsufflation des poumons d'un patient.

11. Dispositif mécanique d'insufflation-exsufflation de la revendication 10, dans lequel l'unité de commande ouvre la deuxième soupape et ferme la première soupape simultanément pour entraîner l'exsufflation des poumons d'un patient lorsqu'une pression inspiratoire maximale est générée par le ventilateur mécanique médical ou autre dispositif approprié destiné à transporter le flux d'air sous pression positive, dans l'unité d'interface patient reliant le ventilateur mécanique médical ou autre dispositif approprié destiné à transporter le flux d'air sous pression positive, et l'unité d'aspiration au patient.

12. Dispositif mécanique d'insufflation-exsufflation de la revendication 10, dans lequel l'unité de commande est configurée pour fermer la deuxième soupape et ouvrir la première soupape simultanément afin d'arrêter l'exsufflation des poumons d'un patient.

13. Dispositif mécanique d'insufflation-exsufflation de la revendication 9, dans lequel l'unité de commande commande le fonctionnement du ventilateur mécanique médical ou autre dispositif approprié destiné à transporter le flux d'air sous pression positive.

14. Dispositif mécanique d'insufflation-exsufflation selon l'une quelconque des revendications 1 à 8, comprenant en outre une unité de commande destinée à commander ledit dispositif mécanique d'insufflation-exsufflation, ayant un microprocesseur comportant des instructions pour exécuter les étapes mises en oeuvre par ordinateur qui consistent :
à ouvrir et fermer de façon sélective la première soupape pour permettre un écoulement d'air sous pression positive à partir du ventilateur mécanique médical ou autre dispositif approprié destiné à transporter le flux d'air sous pression positive, afin de procéder à une insufflation des poumons d'un patient ; et
à ouvrir et fermer de façon sélective la deuxième soupape pour permettre un écoulement d'air sous pression négative afin de procéder à une exsufflation des poumons d'un patient.

15. Dispositif mécanique d'insufflation-exsufflation de la revendication 14, dans lequel l'unité de commande est programmée pour mettre en marche l'unité d'aspiration afin de générer le flux d'air sous pression négative à la demande.

16. Dispositif mécanique d'insufflation-exsufflation de la revendication 14, dans lequel l'unité de commande est programmée pour exécuter l'étape qui consiste à ouvrir la deuxième soupape et fermer la première soupape simultanément après mise en marche de l'unité d'aspiration pour entraîner l'exsufflation.

17. Dispositif mécanique d'insufflation-exsufflation de la revendication 16, dans lequel l'unité de commande est programmée pour exécuter l'étape qui consiste à augmenter un volume courant du flux d'air sous pression positive fourni au patient immédiatement avant l'étape qui consiste à ouvrir la deuxième soupape et fermer la première soupape simultanément.

18. Dispositif mécanique d'insufflation-exsufflation de la revendication 16, dans lequel l'unité de commande est programmée pour exécuter l'étape qui consiste à ouvrir la deuxième soupape et fermer la première soupape simultanément lorsqu'une pression inspiratoire maximale est atteinte chez un patient.

19. Dispositif mécanique d'insufflation-exsufflation de la revendication 18, dans lequel l'unité de commande est programmée pour exécuter l'étape qui consiste à fermer la deuxième soupape et ouvrir la première soupape simultanément après une durée prédéterminée afin d'arrêter l'exsufflation.

20. Dispositif mécanique d'insufflation-exsufflation de la revendication 18, dans lequel l'unité de commande est programmée pour déclencher l'étape qui consiste à ouvrir la deuxième soupape et fermer la première soupape simultanément à partir de l'une : d'une activation d'un bouton de commande, d'une détection d'une forte pression intrathoracique chez un patient et après un intervalle prédéterminé.

21. Dispositif mécanique d'insufflation-exsufflation de la revendication 14, dans lequel l'unité de commande est programmée pour annuler une fonction d'alarme du ventilateur mécanique médical ou autre dispositif approprié destiné à transporter le flux d'air sous pression positive, qui génère le flux d'air sous pression positive afin de procéder à une insufflation des poumons d'un patient.

22. Dispositif mécanique d'insufflation-exsufflation de la revendication 14, dans lequel la première soupape est une membrane à activation pneumatique et l'unité de commande déclenche un mécanisme pneumatique pour générer une augmentation de pression pneumatique afin de fermer sélectivement la première soupape.
